(19) Europäisches Patentamt
European Patent Office
Office européen des brevets

(11) EP 1 406 857 B1

(12) # EUROPÄISCHE PATENTSCHRIFT

(45) Veröffentlichungstag und Bekanntmachung des Hinweises auf die Patenterteilung:
**21.02.2007 Patentblatt 2007/08**

(21) Anmeldenummer: **02754818.9**

(22) Anmeldetag: **03.07.2002**

(51) Int Cl.:
***C07C 211/28*** *(2006.01)* ***A61P 25/04*** *(2006.01)*
***A61P 25/24*** *(2006.01)* ***A61P 25/28*** *(2006.01)*
***A61P 27/00*** *(2006.01)* ***A61K 31/137*** *(2006.01)*

(86) Internationale Anmeldenummer:
**PCT/EP2002/007381**

(87) Internationale Veröffentlichungsnummer:
**WO 2003/004452 (16.01.2003 Gazette 2003/03)**

(54) **SUBSTITUIERTE 1-ARYL-BUT-3-ENYLAMIN- UND 1-ARYL-BUT-2-ENYLAMINVERBINDUNGEN**
SUBSTITUTED 1-ARYL-BUT-3-ENYLAMINE AND 1-ARYL-BUT-2-ENYLAMINE COMPOUNDS
COMPOSES DE 1-ARYL-BUT-3-ENYLAMINES ET 1-ARYL-BUT-2-ENYLAMINES SUBSTITUES

(84) Benannte Vertragsstaaten:
**AT BE BG CH CY CZ DE DK EE ES FI FR GB GR IE IT LI LU MC NL PT SE SK TR**
Benannte Erstreckungsstaaten:
**LT LV RO SI**

(30) Priorität: **05.07.2001 DE 10132747**

(43) Veröffentlichungstag der Anmeldung:
**14.04.2004 Patentblatt 2004/16**

(73) Patentinhaber: **Grünenthal GmbH**
**52078 Aachen (DE)**

(72) Erfinder:
- **SUNDERMANN, Bernd**
  **52066 Aachen (DE)**
- **MAUL, Corinna**
  **52066 Aachen (DE)**
- **BUSCHMANN, Helmut**
  **E-08950 Esplugues de Llobregat (ES)**
- **KÖGEL, Babette-Yvonne**
  **52379 Langerwehe-Hamaich (DE)**

(56) Entgegenhaltungen:
**DE-C- 963 424** **US-A- 3 564 100**
**US-A- 5 811 582**

- **LEDNICER D ET AL: "PARTLY REDUCED BIPHENYLS AS CENTRAL NERVOUS SYSTEM AGENTS 1. 4-ARYLCYCLOHEX-3-ENYLAMINES" JOURNAL OF MEDICINAL CHEMISTRY, AMERICAN CHEMICAL SOCIETY. WASHINGTON, US, Bd. 15, Nr. 12, 1972, Seiten 1235-1238, XP000990795 ISSN: 0022-2623**

Bemerkungen:
Die Akte enthält technische Angaben, die nach dem Eingang der Anmeldung eingereicht wurden und die nicht in dieser Patentschrift enthalten sind.

EP 1 406 857 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft substituierte 1-Aryl-but-3-enylamin- und 1-Aryl-but-2-enylaminverbindungen, Verfahren zu deren Herstellung, Arzneimittel enthaltend diese Verbindungen sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Noziception in letzter Zeit erschienen sind.

Klassische Opioide, wie beispielsweise das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation sowie Toleranzentwicklung auf. Nach weiteren schmerzhemmenden Mitteln wird weltweit geforscht.

1-Aryl-but-3-enylaminverbindung wurde in US 5811582 als analgesische Substanz beschrieben.

Aufgabe der vorliegenden Erfindung war es daher, neue Verbindungen zur Verfügung zu stellen, die sich insbesondere als pharmazeutische Wirkstoffe in Arzneimitteln eignen.

Diese Wirkstoffe sollen sich insbesondere zur Schmerzbekämpfung eignen. Des weiteren sollen sich die Wirkstoffe auch zur Behandlung von Depressionen, Hypotension, Hypertension, seniler Demenz, Morbus Alzheimer, allgemeinen kognitiven Dysfunktionen, Tinnitus, Schwerhörigkeit, Epilepsie, Fettsucht, Kachexie, Haminkontinenz oder zur Anxiolyse oder Diurese eignen.

Erfindungsgemäß wird diese Aufgabe durch die Bereitstellung substituierter 1-Aryl-but-3-enylamin- und 1-Aryl-but-2-enylaminverbindungen der nachstehenden allgemeinen Formel I gelöst, die eine ausgeprägte analgetische Wirkung aufweisen und die sich insbesondere auch zur Behandlung von Depressionen, Hypotension, Hypertension, seniler Demenz, Morbus Alzheimer, allgemeinen kognitiven Dysfunktionen, Tinnitus, Schwerhörigkeit, Epilepsie, Fettsucht, Kachexie, Harninkontinenz oder zur Anxiolyse oder Diurese eignen.

Ein Gegenstand der vorliegenden Erfindung sind daher substituierte 1-Aryl-but-3-enylamin- und 1-Aryl-but-2-enylaminverbindungen der allgemeinen Formel I,

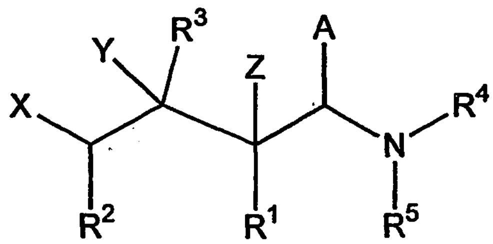

I

worin

$R^1$ und $R^2$, gleich oder verschieden, für einen $C_{1-6}$ Alkyl-Rest stehen oder zusammen als einen $(CH_2)_{2-6}$-Ring bilden, der auch mit wenigstens einem gegebenenfalls wenigstens einfach substituierten Aryl- oder Heteroaryl-Rest substituiert oder benzokondensiert sein kann, vorzugsweise zusammen als einen $(CH_2)_{2-6}$-Ring bilden, der auch mit wenigstens einem gegebenenfalls wenigstens einfach substituierten Aryl- oder Heteroaryl-Rest substituiert oder benzokondensiert sein kann, besonders bevorzugt zusammen einen Cyclohexyl-Ring bilden, der auch mit einem gegebenenfalls wenigstens einfach substituierten Phenyl-Rest substituiert sein kann,

$R^3$ für einen $C_{3-6}$-Alkyl-, einen $C_{3-7}$-Cycloalkyl-, einen gegebenenfalls wenigstens einfach substituierten Aryl- oder Heteroaryl-Rest oder für einen über eine $C_{1-3}$-Alkylen-Gruppe gebundenen, gegebenenfalls wenigstens einfach substituierten Aryl- oder Heteroaryl-Rest, vorzugsweise für einen gegebenenfalls wenigstens einfach substituierten Aryl-Rest oder für einen über eine $C_{1-3}$-Alkylen-Gruppe gebundenen, gegebenenfalls wenigstens einfach substituierten Aryl-Rest, besonders bevorzugt für einen gegebenenfalls wenigstens einfach substituierten Phenyl-, Benzyl- oder Phenethyl-Rest, steht,

$R^4$ und $R^5$, gleich oder verschieden, für einen $C_{1-6}$-Alkyl-, einen $C_{3-7}$-Cycloalkyl-, einen Phenyl-, einen Benzyl- oder

einen Phenethyl-Rest stehen oder $R^4$ und $R^5$ zusammen einen $(CH_2)_{3-6}$- oder $-CH_2-CH_2-O-CH_2-CH_2$-Ring bilden, vorzugsweise, gleich oder verschieden, für einen $C_{1-6}$-Alkyl-Rest stehen oder zusammen einen $-(CH_2)_5$- oder $-CH_2-CH_2-O-CH_2-CH_2$-Ring bilden, besonders bevorzugt, gleich oder verschieden, für einen $C_{1-2}$-Alkyl-Rest stehen,

X und Y oder Y und Z zusammen für eine Bindung, vorzugsweise X und Y zusammen für eine Bindung stehen,

A für einen gegebenenfalls wenigstens einfach substituierten Aryl- oder Heterorary-Rest, vorzugsweise für einen gegebenenfalls wenigstens einfach substituierten Aryl-Rest, besonders bevorzugt für einen gegebenenfalls wenigstens einfach substituierten Phenyl-Rest, steht,

in Form ihrer Racemate, Diasteromeren oder Enantiomeren als freie Base oder eines entsprechenden physiologisch verträglichen Salzes.

Der Ausdruck "Alkyl-Rest" umfaßt im Sinne der vorliegenden Erfindung geradkettige oder verzweigte Kohlenwasserstoffreste. Vorzugsweise sind die Alkyl-Reste ausgewählt aus der Gruppe bestehend aus Methyl, Ethyl, Propyl, Isopropyl, n-Butyl, sek.-Butyl, tert.-Butyl, n-Pentyl, Neopentyl und n-Hexyl.

Der Ausdruck "Aryl-Rest" umfaßt im Sinne der vorliegenden Erfindung aromatische Kohlenwasserstoffe. Sofern der Aryl-Rest mehr als einen Substituenten aufweist, können diese gleich oder verschieden sein. Bevorzugt ist der Aryl-Rest ein gegebenenfalls wenigstens einfach mit F, Cl, Br, $CHF_2$, $CF_3$, OH, $OCF_3$, $OR^6$, $NR^7R^8$, $SR^6$, Phenyl, $SO_2-CH_3$, $SO_2-CF_3$, $C_{1-6}$-Alkyl, CN, $COOR^6$, $CONR^7R^8$ substituierter Phenyl-Rest, worin

$R^6$ für einen $C_{1-6}$-Alkyl-, einen Phenyl-, einen Benzyl- oder einen Phenethyl-Rest steht,

$R^7$ und $R^8$, gleich oder verschieden, für H, einen $C_{1-6}$-Alkyl-, einen Phenyl-, einen Benzyl- oder einen Phenethyl-Rest stehen.

Es können auch zwei Substituenten des Aryl-Restes einen gesättigten oder ungesättigten, gegebenenfalls Heteroatome aufweisenden Kohlenwasserstoff Ring am Aryl-Rest, vorzugweise einen $-OCH_2O-$, $-OCH_2CH_2O-$, -CH=CHO-, $-CH=C(CH_3)O-$ oder $-(CH_2)_4$-Ring bilden. Ebenfalls bevorzugt ist ein substituierter Phenyl-Rest, dessen zwei Substituenten einen $-OCH_2O-$, $-OCH_2CH_2O-$, -CH=CHO-, $-CH=C(CH_3)O-$ oder $-(CH_2)_4$-Ring bilden.

Der Ausdruck "Cycloalkyl-Rest" umfaßt im Sinne der vorliegenden Erfindung gesättigte zyklische Kohlenwasserstoffreste oder Alkyl-Reste, die eine solche Teilstruktur aufweisen. Vorzugsweise sind die Cycloalkyl-Reste ausgewählt aus der Gruppe bestehend aus Cyclopropyl, Cyclopropylmethyl, Methylcyclopropyl, Cyclobutyl, 1-Cyclopropylethyl, 2-Cyclopropylethyl, Cyclopentyl, Cyclopentylmethyl, Cyclohexyl und Cycloheptyl.

Der Ausdruck "Heteroaryl-Rest" steht im Sinne der vorliegenden Erfindung für einen vorzugsweise 5- oder 6-gliedrigen zyklischen aromatischen Rest, der ein oder mehr Heteroatome aufweist. Sofern der Heteroaryl-Rest mehr als ein Heteroatom aufweist, können diese gleich oder verschieden sein. Bevorzugt sind die Heteroatome ausgewählt aus der Gruppe bestehend aus Stickstoff, Sauerstoff und Schwefel.
Sofern der Heteroaryl-Rest mehr als einen Substituenten aufweist, können diese gleich oder verschieden sein.

Bevorzugt ist der Heteroaryl-Rest ein gegegebenfalls wenigstens einfach substituierter Furanyl-, Thiophenyl-, Pyrrolyl-, Pyridinyl-, Pyrimidinyl-, Chinolinyl-, Isochinolinyl-, Phthalazinyl- oder Chinazolinyl-Rest.

Vorzugsweise kann der Heteroaryl-Rest einfach oder mehrfach mit einem Substituenten ausgewählt aus der Gruppe bestehend aus F, Cl, Br, $CHF_2$, $CF_3$, OH, $OCF_3$, $OR^6$, $NR^7R^8$, $SR^6$, Phenyl, $SO_2-CH_3$, $SO_2-CF_3$, $C_{1-6}$-Alkyl, CN, $COOR^6$ und $CONR^7R^8$ substituiert sein, worin die Reste $R^6$, $R^7$ und $R^8$ die oben angegebene Bedeutung haben.

Es können auch zwei Substituenten des Heteroaryl-Restes einen gesättigten oder ungesättigten, ggf. Heteroatome aufweisenden Kohlenwasserstoff-Ring am Heteroaryl-Rest, vorzugweise einen $-OCH_2O-$, $-OCH_2CH_2O-$, -CH=CHO-, $-CH=C(CH_3)O-$ oder $-(CH_2)_4$-Ring bilden.

Ganz besonders bevorzugt sind folgende substituierte 1-Aryl-but-3-enylamin- und 1-Aryl-but-2-enylaminverbindungen sowie deren entsprechende physiologisch verträglichen Salze, vorzugsweise deren Hydrochloride:

Dimethyl-[phenyl-(2-phenyl-cyclohex-1-enyl)-methyl]-amin,
{[2-(4-Chlor-phenyl)-cyclohex-1-enyl]-phenyl-methyl}-dimethyl-amin,
[(2-Benzyl-cyclohex-1-enyl)-phenyl-methyl]-dimethyl-amin,
{[2-(4-Fluor-3-methyl-phenyl)-cyclohex-1-enyl]-phenyl-methyl}-dimethyl-amin,
Dimethyl-[phenyl-(2-o-tolyl-cyclohex-1-enyl)-methyl]-amin,
[(2-Cyclopentyl-cyclohex-1-enyl)-phenyl-methyl]-dimethyl-amin,
Dimethyl-[phenyl-(2-m-tolyl-cyclohex-1-enyl)-methyl]-amin,
(Bicyclohexyl-1-en-2-yl-phenyl-methyl)-dimethyl-amin,
{[2-(4-Fluor-phenyl)-cyclohex-1-enyl]-phenyl-methyl}-dimethyl-amin,

Dimethyl-[(2-phenethyl-cyclohex-1-enyl)-phenyl-methyl]-amin,
{[2-(3-Methoxy-phenyl)-cyclohex-1-enyl]-phenyl-methyl}-dimethyl-amin,
Dimethyl-{phenyl-[2-(3-phenyl-propyl)-cyclohex-1-enyl]-methyl}-amin,
{[2-(2-Chlor-benzyl)-cyclohex-1-enyl]-phenyl-methyl}-dimethyl-amin,
{[2-(4-Fluor-benzyl)-cyclohex-1-enyl]-phenyl-methyl}-dimethyl-amin,
{[2-(3-Methoxy-benzyl)-cyclohex-1-enyl]-phenyl-methyl}-dimethyl-amin,
{[2-(3-Fluor-benzyl)-cyclohex-1-enyl]-phenyl-methyl}-dimethyl-amin,
{[2-(2-Methoxy-benzyl)-cyclohex-1-enyl]-phenyl-methyl}-dimethyl-amin,
{[2-(3,5-Difluor-benzyl)-cyclohex-1-enyl]-phenyl-methyl}-dimethyl-amin,
{[2-(2-Fluor-benzyl)-cyclohex-1-enyl]-phenyl-methyl}-dimethyl-amin,
{[2-(2-Chlor-benzyl)-cyclohex-1-enyl]-phenyl-methyl}-dimethyl-amin,
{[2-(3-Fluor-benzyl)-cyclohex-1-enyl]-phenyl-methyl}-dimethyl-amin,
Dimethyl-{phenyl-[2-(3-trifluormethyl-benzyl)-cyclohex-1-enyl]-methyl}-amin,
Dimethyl-[(2-phenethyl-cyclohex-1-enyl)-phenyl-methyl]-amin,
3-[6-(Dimethylamino-phenyl-methyl)-cyclohex-1-enyl]-phenol,
Dimethyl-{phenyl-[2-(4-trifluormethylphenyl)-cyclohex-1-enyl]-methyl}-amin,
2-Chlor-5-[6-(dimethylamino-phenyl-methyl)-cyclohex-1-enyl]-phenol,
{[2-(4-Methoxy-phenyl)-cyclohex-2-enyl]-phenyl-methyl}-dimethyl-amin,
{[2-(4-Chlor-phenyl)-cyclohex-1-enyl]-phenyl-methyl}-dimethyl-amin und
Dimethyl-[(2-phenyl-cyclohex-1-enyl)-(4-trifluormethyl-phenyl)-methyl]-amin.

Die erfindungsgemäßen substituierten 1-Aryl-but-3-enylamin- und 1-Aryl-but-2-enylaminverbindungen der allgemeinen Formel I oder deren entsprechende physiologisch verträgliche Salze können jeweils in Form ihrer Racemate, ihrer reinen Enantiomeren, ihrer reinen Diastereomeren oder in Form eines Gemisches aus wenigstens zwei der vorstehend genannten Stereoisomeren vorliegen. Ebenso können die erfindungsgemäßen substituierten 1-Aryl-but-3-enylamin- und 1-Aryl-but-2-enylaminverbindungen der allgemeinen Formel I jeweils auch in Form von Mischungen ihrer Enantiomeren oder Diastereomeren, insbesondere in Form ihrer cis/trans-Diastereomeren vorliegen. Diese Mischungen können die jeweiligen Stereoisomeren in jedem beliebigen Mischungsverhältnis aufweisen.

Ein weiterer Gegenstand der vorliegenden Erfindung ist ein Verfahren zur Herstellung von substituierten 1-Aryl-but-3-enylamin- und 1-Aryl-but-2-enylaminverbindungen der oben angegebenen allgemeinen Formel I, gemäß dem wenigstens eine Mannich-Base der allgemeinen Formel II,

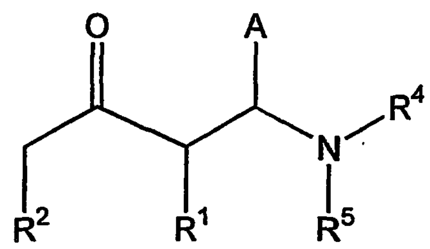

II

worin $R^1$, $R^2$, $R^4$, $R^5$ und A die oben angegebene Bedeutung haben, mit wenigstens einer metallorganischen Verbindung der allgemeinen Formel $R^3$-B, worin B für MgCl, MgBr, Mgl oder Li steht und $R^3$ die oben angegebene Bedeutung hat, zu einem Alkohol der allgemeinem Formel III,

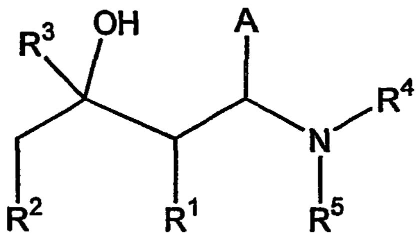

III

umgesetzt wird, worin die Reste $R^1$ bis $R^5$ und A die oben angegebene Bedeutung haben und diese gegebenenfalls nach üblichen Methoden gereinigt und/oder gegebenfalls nach üblichen Methoden isoliert, und mit einer geeigneten Säure ggf. in Gegenwart eines geeigneten Lösungsmittels zu wenigstens einer Verbindung der oben angegebenen allgemeinen Formel I umgesetzt wird.

Das Verfahren zur Herstellung der Alkohole der allgemeinen Formel III ist beispielsweise auch in der DE 19915 601 A1 beschrieben. Die entsprechende Literaturbeschreibung wird hiermit als Referenz eingeführt und gilt als Teil der Offenbarung.

In einer bevorzugten Ausführungsform des erfindungsgemäßen Verfahrens wird als geeignete Säure für die Umsetzung des Alkohols der allgemeinen Formel III zu der Verbindung der allgemeinen Formel I eine Protonensäure, eine Lewissäure oder deren Mischung eingesetzt.
Als Protonensäure kommt bevorzugt Bromwasserstoff, Chlorwasserstoff, Ameisensäure, als Lewissäure bevorzugt Trimethylsilyliodid oder Chlortrimethylsilan zum Einsatz.

Als geeignetes Lösungsmittel kann während der Umsetzung des Alkohols der allgemeinen Formel III zu der Verbindung der allgemeinen Formel I Wasser zum Einsatz kommen. Des weiteren können auch organische Lösungsmittel, wie beispielsweise Acetonitril, gegebenfalls auch im Gemisch mit Wasser zum Einsatz kommen.

Die Temperatur kann während der Umsetzung des Alkohols der allgemeinen Formel III mit der Säure über einen weiten Bereich variieren. Bevorzugt wird die Umsetzung bei einer Temperatur von 5 bis 150 °C, besonders bevorzugt bei einer Temperatur von 10 bis 130 °C, ganz besonders bevorzugt bei einer Temperatur von 15 bis 120 °C durchgeführt.

Das erfindungsgemäße Verfahren kann auch in semi- oder vollautomatisierter Form als Parallelsynthese einer Gruppe von erfindungsgemäßen 1-Aryl-but-3-enylamin- und 1-Aryl-but-2-enylaminverbindungen der allgemeinen Formel I durchgeführt werden.

Die erfindungsgemäßen substituierten 1-Aryl-but-3-enylamin- und 1-Ary1-but-2-enylaminverbindungen der allgemeinen Formel I können nach dem erfindungsgemäßen Verfahren sowohl in Form ihrer freien Base als auch in Form eines Salzes isoliert werden.

Sowohl die Überführung der freien Base der erfindungsgemäßen Verbindungen der allgemeinen Formel I in ein entsprechendes physiologisch verträgliches Salz als auch die Freisetzung der freien Base aus dem entsprechenden physiologisch verträglichen Salz kann nach üblichen, dem Fachmann bekannten Methoden erfolgen.

Die Überführung der freien Base einer erfindungsgemäßen Verbindung der allgemeinen Formel I in ihr entsprechendes physiologisch verträgliches Salz kann beispielsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure erfolgen.
Die freie Base der jeweiligen erfindungsgemäßen Verbindung der allgemeinen Formel I kann auch mit der freien Säure oder einem Salz eines Zuckerersatzstoffes, wie z.B. Saccharin, Cyclamat oder Acesulfam, in das entsprechende physiologisch verträgliche Salz übergeführt werden.

Die Überführung der freien Base einer erfindungsgemäßen Verbindung der allgemeinen Formel I in ihr entsprechendes Hydrochlorid kann bevorzugt auch durch Versetzen der in einem geeigneten organischen Lösungsmittel, wie z.B. Butan-2-on (Methylethylketon), gelösten erfindungsgemäßen Verbindung der allgemeinen Formel I als freie Base mit Trimethylsilylchlorid (TMSCl) erhalten werden.

Sofern eine Auftrennung der erfindungsgemäßen substituierten 1-Aryl-but-3-enylamin- und 1-Aryl-but-2-enylaminverbindungen der allgemeinen Formel I in ihre verschiedenen Enantiomeren und/oder Diastereomeren gewünscht ist, kann diese nach üblichen, dem Fachmann bekannten Verfahren erfolgen. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren,

etwa (+)-Weinsäure, (-)-Weinsäure oder (+)-10-Camphersulfonsäure, gebildete diastereomere Salze voneinander getrennt werden.

Die erfindungsgemäßen substituierten 1-Aryl-but-3-enylamin- und 1-Aryl-but-2-enylaminverbindungen der allgemeinen Formel I sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße substituierte 1-Aryl-but-3-enylamin-oder 1-Aryl-but-2-enylaminverbindung der allgemeinen Formel I sowie gegebenenfalls physiologisch verträgliche Hilfsstoffe enthalten.

Vorzugsweise eignen sich die erfindungsgemäßen Arzneimittel zur Bekämpfung von Schmerzen oder zur Behandlung von Depressionen, Hypotension, Hypertension, seniler Demenz, Morbus Alzheimer, allgemeinen kognitiven Dysfunktionen, Tinnitus, Schwerhörigkeit, Epilepsie, Fettsucht, Kachexie, Haminkontinenz, zur Anxiolyse oder zur Diurese.

Die Verwendung wenigstens einer substituierten 1-Aryl-but-3-enylamin- oder 1-Aryl-but-2-enylaminverbindung der allgemeinen Formel I zur Herstellung eines Arzneimittels zur Bekämpfung von Schmerzen, zur Behandlung von Depressionen, Hypotension, Hypertension, seniler Demenz, Morbus Alzheimer, allgemeinen kognitiven Dysfunktionen, Tinnitus, Schwerhörigkeit, Epilepsie, Fettsucht, Kachexie, Harninkontinenz, zur Anxiolyse oder zur Diurese istebenfalls Gegenstand der vorliegenden Erfindung.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einer erfindungsgemäßen substituierten 1-Aryl-but-3-enylamin- oder 1-Aryl-but-2-enylaminverbindung der allgemeinen Formel I enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel I auch verzögert freisetzen.

Die Herstellung der erfindungsgemäßen Arzneimittel kann mit Hilfe von üblichen, dem Fachmann bekannten Mitteln, Vorrichtungen, Methoden und Verfahren erfolgen, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen substituierten 1-Aryl-but-3-enylamin- oder 1-Aryl-but-2-enylaminverbindung der allgemeinen Formel I kann variieren und ist beispielsweise abhängig vom Gewicht oder dem Alter des Patienten sowie von der Applikationsart, der Indikation und dem Schweregrad der Erkrankung. Üblicherweise werden 0,005 bis 500 mg/kg, vorzugsweise 0,05 bis 5 mg/kg Körpergewicht des Patienten wenigstens einer erfindungsgemäßen substituierten 1-Aryl-but-3-enylamin- oder 1-Aryl-but-2-enylaminverbindung der allgemeinen Formel I appliziert.

**Pharmakologische Untersuchungen:**

**Analgesieprüfung im Writhing-Test an der Maus**

Die Untersuchung auf analgetische Wirksamkeit wurde im Phenylchinoninduzierten Writhing an der Maus (modifiziert nach I.C. Hendershot und J. Forsaith (1959) J. Pharmacol. Exp. Ther. 125, 237-240) durchgeführt.
Dazu wurden männliche NMRI-Mäuse mit einem Gewicht von 25 bis 30 g verwendet. Gruppen von 10 Tieren pro Dosis einer Verbindung erhielten 10 Minuten nach intravenöser Gabe der Prüfverbindungen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhielten. Alle Verbindungen wurden in der Standarddosierung von 10 mg/kg getestet. Die prozentuale Hemmung (%Hemmung) der Writhingreaktion durch eine erfindungsgemäße Verbindung der allgemeinen Formel I wurde nach folgender Formel berechnet:

$$\% \text{ Hemmung} = 100 - \frac{\text{Writhingreaktionen der behandelten Tiere}}{\text{Writhingreaktionen der Kontrolltiere}} * 100$$

Im folgenden wird die Erfindung anhand von Beispielen erläutert. Diese Erläuterungen sind lediglich beispielhaft.

**Beispiele:**

Die Ausbeuten der erfindungsgemäßen Beispielverbindungen wurden nicht optimiert.

Alle Temperaturen sind unkorrigiert.

Die Herstellung der in den nachfolgend angegebenen Beispielen jeweils eingesetzten Alkohole wurde nach Verfahren durchgeführt, wie sie in der DE 199 15 601 beschrieben sind. Die entsprechende Literaturbeschreibung wird hiermit als Referenz eingeführt und gilt als Teil der Offenbarung.

**Allgemeine Arbeitsvorschrift 1**

Auf einem Syntheseroboter (Syro II, Multisyntech) wurden je 10 mg des jeweiligen Alkohols der allgemeinen Formel III mit 2 ml Ameisensäure versetzt und für zwei Stunden auf 90°C erhitzt. Nach der Reaktion wurde das Reaktionsgemisch in einer Vakuumzentrifuge eingeengt. Die Substanzen wurden mittels ESI-MS analysiert. Die jeweils eingesetzten Alkohole sowie die erhaltenen beispielgemäßen Verbindungen der allgemeinen Formel I sind in der nachfolgenden Tabelle 1 wiedergegeben.

Tabelle 1:

| Beispiel | eingesetzter Alkohol der allgemeinen Formel III | 1-Aryl-but-3-enylamin-bzw. 1-Aryl-but-2-enylaminverbindung der allgemeinen Formel I |
|---|---|---|
| | | |
| 1 | 2-(Dimethylamino-phenylmethyl)-1-phenyl-cyclohexanol | Dimethyl-[phenyl-(2-phenyl-cyclohex-1-enyl)-methyl]-amin |
| 2 | 1-(4-Chlor-phenyl)-2-(dimethylamino-phenyl-methyl)-cyclohexanol | {[2-(4-Chlor-phenyl)-cyclohex-1-enyl]-pheny1-methyl}-dimethyl-amin |
| 3 | 1-Benzyl-2-(dimethylamino-phenyl-methyl)-cyclohexanol | [(2-Benzyl-cyclohex-1-enyl)-phenyl-methyl]-dimethyl-amin |
| 4 | 2-(Dimethylamino-phenyl-methyl)-1-(4-fluor-3-methyl-phenyl)-cyclohexanol | {[2-(4-Fluor-3-methyl-phenyl)-cyclohex-1-enyl]-phenyl-methyl}-dimethyl-amin |
| 5 | 2-(Dimethylamino-phenyl-methyl)-1-o-tolyl-cyclohexanol | Dimethyl-[phenyl-(2-o-tolyl-cyclohex-1-enyl)-methyl]-amin |
| 6 | 1-Cyclopentyl-2-(dimethylamino-phenyl-methyl)-cyclohexanol | [(2-Cyclopentyl-cyclohex-1-enyl)-phenyl-methyl]-dimethyl-amin |
| 7 | 2-(Dimethylamino-phenyl-methyl)-1-m-tolyl-cyclohexanol | Dimethyl-[phenyl-(2-m-tolyl-cyclohex-1-enyl)-methyl]-amin |

(fortgesetzt)

| Beispiel | eingesetzter Alkohol der allgemeinen Formel III | 1-Aryl-but-3-enylamin-bzw. 1-Aryl-but-2-enylaminverbindung der allgemeinen Formel I |
|---|---|---|
| | | |
| 8 | 2-(Dimethylamino-phenyl-methyl)-bicyclohexyl-1-ol | (Bicyclohexyl-1-en-2-yl-phenyl-methyl)-dimethyl-amin |
| 9 | 2-(Dimethylamino-phenyl-methyl)-1-(4-fluor-phenyl)-cyclohexanol | {[2-(4-Fluor-phenyl)-cyclohex-1-enyl]-phenyl-methyl}-dimethyl-amin |
| 10 | 2-(Dimethylamino-phenyl-methyl)-1-phenethyl-cyclohexanol | Dimethyl-[(2-phenethyl-cyclohex-1-enyl)-phenyl-methyl]-amin |
| 11 | 2-(Dimethylamino-phenyl-methyl)-1-(3-methoxy-phenyl)-cyclohexanol | {[2-(3-Methoxy-phenyl)-cyclohex-1-enyl]-phenyl-methyl}-dimethyl-amin |
| 12 | 2-(Dimethylamino-phenyl-methyl-)-1-(3-phenyl-propyl)-cyclohexanol | Dimethyl-{phenyl-[2-(3-phenyl-propyl)-cyclohex-1-enyl]-methyl}-amin |
| 13 | 1-(2-Chlor-benzyl)-2-dimethylamino-phenyl-methyl)-cyclohexanol | ([2-(2-Chlor-benzyl)-cyclohex-1-enyl]-phenyl-methyl}-dimethyl-amin |
| 14 | 2-(Dimethylamino-phenyl-methyl)-1-(4-fluor-benzyl)-cyclohexanol | {[2-(4-Fluor-benzyl)-cyclohex-1-enyl]-phenyl-methyl}-dimethyl-amin |
| 15 | 2-(Dimethylamino-phenyl-methyl)-1-(3-methoxy-benzyl)-cyclohexanol | {[2-(3-Methoxy-benzyl)-cyclohex-1-enyl]-phenyl-methyl}-dimethyl-amin |
| 16 | 2-(Dimethylamino-phenyl-methyl)-1-(3-fluor-benzyl)-cyclohexanol | {[2-(3-Fluor-benzyl)-cyclohex-1-enyl]-phenyl-methyl}-dimethyl-amin |
| 17 | 2-(Dimethylamino-phenyl-methyl)-1-(2-methoxy-benzyl)-cyclohexanol | {[2-(2-Methoxy-benzyl)-cyclohex-1-enyl]-phenyl-methyl)-dimethyl-amin |
| 18 | 1-(3,5-Difluor-benzyl)-2-(dimethylamino-phenyl-methyl)-cyclohexanol | {[2-(3,5-Difluor-benzyl)-cyclohex-1-enyl]-phenyl-methyl}-dimethyl-amin |

**Allgemeine Arbeitsvorschrift 2**

Der jeweils eingesetzte Alkohol der allgemeinen Formel III wurde mit wäßriger HBr, (47 Massen-%; circa 10 ml pro mmol Alkohol) für fünf Stunden zum Rückfluß erhitzt. Anschließend wurde unter Eiskühlung mit Natronlauge (32 Massen-%) basisch gestellt und dreimal mit Dichlormethan (circa 10 ml pro mmol Alkohol) extrahiert. Die vereinigten Extrakte wurden über Natriumsulfat getrocknet, filtriert und eingeengt.

Die so erhaltenen Rohprodukte wurden ohne weitere Aufreinigung in die entsprechenden Hydrochloride übergeführt. Dazu wurde das jeweilige Rohprodukt in ca. 10 ml 2-Butanon je Gramm freie Base gelöst, ein halbes Moläquivalent Wasser, gefolgt von 1,1 Moläquivalenten Chlortrimethylsilan zugegeben und über Nacht gerührt. Anschließend wurden bereits ausgefallene Hydrochloride abfiltriert oder die Hydrochloride wurden durch Zugabe von Diethylether und/oder n-Hexan ausgefällt und anschließend im Vakuum getrocknet.

Die jeweils eingesetzten Alkohole, die erhaltenen Hydrochloride der beispielgemäßen Verbindungen der allgemeinen Formel I, deren Ausbeute und ggf. das zur Fällung des Hydrochlorids eingesetzte Lösungsmittel sind in der nachfolgenden Tabelle 2 wiedergegeben.

Tabelle 2:

| Beispiel | eingesetzter Alkohol | 1-Aryl-but-3-enylamin-bzw. 1-Aryl-but-2-enylaminverbindung der allgemeinen Formel I | Ausbeute Hydrochloriding | Ausfällung mit |
|---|---|---|---|---|
| | | | | |
| 19 | 2-(Dimethylamino-phenyl-methyl)-1-(2-fluor-benzyl)-cyclohexanol | ([2-(2-Fluor-benzyl)-cyclohex-1-enyl]-phenyl-methyl}-dimethyl-amin-Hydrochlorid | 1,31 | - |
| 20 | 1-(2-Chlor-benzyl)-2-(dimethylamino-phenyl-methyl)-cyclohexanol | {[2-(2-Chlor-benzyl)-cyclohex-1-enyl]-phenyl-methyl}-dimethyl-amin-Hydrochlorid | 2,17 | Dieethylether |
| 21 | 2-(Dimethylamino-phenyl-methyl)-1-(3-fluor-benzyl)-cyclohexanol | {[2-(3-Fluor-benzyl)-cyclohex-1-enyl]-phenyl-methyl)-dimethyl-amin-Hydrochlorid | 0,89 | - |
| 22 | 2-(Dimethylamino-phenyl-methyl)-1-(3-trifluormethyl-benzyl)-cyclohexanol | Dimethyl-{phenyl-[2-(3-trifluormethyl-benzyl)-cyclohex-1-enyl]-methyl}-amin-Hydrochlorid | 2,64 | - |
| 23 | 2-(Dimethylamino-phenyl-methyl)-1-phenethyl-cyclohexanol | Dimethyl-[(2-phenethyl-cyclohex-1-enyl)-phenyl-methyl]-amin-ydrochlorid | 1,45 | - |
| 25 | 2-(Dimethylamino-phenyl-methyl)-1-(4-trifluormethyl-phenyl)-cyclohexanol | Dimethyl-{phenyl-[2-(4-trifluormethylphenyl)-cyclohex-1-enyl]-methyl}-amin | 0,75 | Dieethylether |
| 26 | 2-Chlor-5-[2-(dimethylamino-phenyl-methyl)-1-hydroxy-cyclohexyl]-phenol | 2-Chlor-5-[6-(dimethylamino-phenyl-methyl)-cyclohex-1-enyl]-phenol | 0,63 | - |
| 28 | 1-(4-Chlor-phenyl)-2-(dimethylamino-phenyl-methyl)-cyclohexanol | ([2-(4-Chlor-phenyl)-cyclohex-1-enyl]-phenyl-methyl)-dimethyl-amin Hydrochlorid | 1,28 | Dieethylether |
| 29 | 2-[Dimethylamino-(4-trifluormethyl-phenyl)-methyl]-1-phenyl-cyclohexanol | Dimethyl-[(2-phenyl-cyclohex-1-enyl)-(4-trifluormethyl-phenyl)-methyl]-amin Hydrochlorid | 1,11 | n-Hexan |

**Beispiel 24:**

**Eliminierung mit Trimethylsilyliodid**

Der Alkohol 3-[2-Dimethylamino-phenyl-methyl)-1-hydroxy-cyclohexyl]-phenol wurde in einer gesättigten Lösung von Natriumiodid in Acetonitril (ca. 3 ml pro mmol Alkohol) gelöst bzw. suspendiert, drei Moläquivalente Trimethylchlorsilan zugetropft und über Nacht gerührt.

Zur Aufarbeitung wurde mit gesättigter Natriumhydrogencarbonatlösung auf pH 8 eingestellt und dann soviel Natriumthiosulfatlösung (ca. 0,1 M) zugegeben, bis eine weitgehend klare und farblose Lösung erhalten wurde. Es wurde dreimal mit Diethylether (ca. 10 ml pro mmol Alkohol) extrahiert, die vereinigten Extrakte über Natriumsulfat getrocknet, filtriert und eingeengt.
Das so erhaltene Rohprodukt wurden ohne weitere Aufreinigung in das Hydrochlorid übergeführt. Dazu wurde das Rohprodukt in ca. 10 ml Aceton je Gramm Base gelöst, 1,1 Moläquivalente Salzsäure (32 Gew-%) zugegeben und über Nacht gerührt. Anschließend wurde das ausgefallene 3-[6-(Dimethylaminophenyl-methyl)-cyclohex-1-enyl]-phenol-Hydrochlorid abfiltriert und im Vakuum getrocknet.

**Beispiel 27:**

Der Akohol 2-(Dimethylamino-phenyl)-1-(4-methoxy-phenyl)-cyclohexanol wurde in ca. 10 ml 2-Butanon je Gramm Alkohol gelöst, ein halbes Moläquivalent Wasser, gefolgt von 1,1 Moläquivalenten Chlortrimethylsilan zugegeben und über Nacht gerührt. Das ausgefallene {[2-(4-Methoxy-phenyl)-cyclohex-2-enyl]-phenyl-methyl)-dimethylamin-Hydrochlorid wurde abfiltriert, die entsprechende Base freigesetzt und chromatographisch aufgereinigt (Kieselgel, Hexan/Ethylacetat 3:2). Anschließend wurde das aufgereinigte {[2-(4-Methoxyphenyl)-cyclohex-2-enyl]-phenyl-methyl}-dimethylamin wie oben beschrieben als Hydrochlorid gefällt.

**Pharmakologische Untersuchungen:**

Die vertiefte Untersuchung der erfindungsgemäßen Verbindungen der allgemeinen Formel I auf analgetische Wirksamkeit wurde im Phenylchinoninduzierten Writhing an der Maus durchgeführt, wie obenstehend beschrieben.
Die untersuchten erfindungsgemäßen Verbindungen zeigten eine analgetische Wirkung.
Die Ergebnisse ausgewählter Writhing-Untersuchungen sind in der nachfolgenden Tabelle 3 zusammengefaßt.

**Tabelle 3:** Analgesieprüfung im Writhing-Test an der Maus

| Beispiel | 1-Aryl-but-3-enylamin-bzw. 1-Aryl-but-2-enylaminverbindung der allgemeinen Formel I | %Hemmung der Writhingreaktion* |
|---|---|---|
| 19 | {[2-(2-Fluor-benzyl)-cyclohex-1-enyl]-phenyl-methyl}-dimethyl-amin | 87% (10 mg/kg) |
| 20 | {[2-(2-Chlor-benzyl)-cyclohex-1-enyl]-phenyl-methyl)-dimethyl-amin | 63% (10 mg/kg) |
| 21 | {[2-(3-Fluor-benzyl)-cyclohex-1-enyl]-phenyl-methyl}-dimethyl-amin | 67% (10 mg/kg) |
| 22 | Dimethyl-{phenyl-[2-(3-trifluormethyl-benzyl)-cyclohex-1-enyl]-methyl}-amin | 46% (21,5 mg/kg) |
| 23 | Dimethyl-[(2-phenethyl-cyclohex-1-enyl)-phenyl-methyl]-amin | 67%(10 mg/kg) |
| 24 | 3-[6-(Dimethylamino-phenyl-methyl)-cyclohex-1-enyl]-phenol Hydrochlorid | 76% (10 mg/kg) |
| 27 | {[2-(4-Methoxy-phenyl)-cyclohex-2-enyl]-phenyl-methyl}-dimethyl-amin Hydrochlorid | 47% (10 mg/kg) |
| 28 | {[2-(4-Chlor-phenyl)-cyclohex-1-enyl]-phenyl-methyl}-dimethyl-amin Hydrochlorid | 71 % (10 mg/kg) |
| 29 | Dimethyl-[(2-phenyl-cyclohex-1-enyl)-(4-trifluormethyl-phenyl)-methyl]-amin Hydrochlorid | 40% (10 mg/kg) |
| * Die Dosierung bei intravenöser Applikation ist in Klammem angegeben. | | |

**Patentansprüche**

1. Substituierte 1-Aryl-but-3-enylamin- und 1-Aryl-but-2-enylaminverbindungen der allgemeinen Formel I,

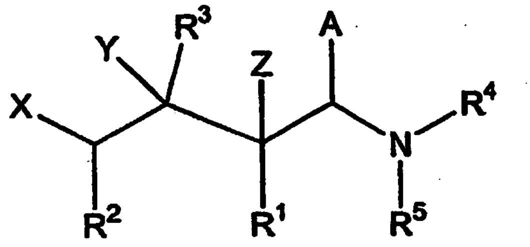

I

worin

$R^1$ und $R^2$, gleich oder verschieden, für einen $C_{1-6}$-Alkyl-Rest stehen oder zusammen als $(CH_2)_{2-6}$ einen Ring bilden, der auch mit wenigstens einem gegebenenfalls wenigstens einfach substituierten Aryl- oder Heteroaryl-Rest substituiert oder benzokondensiert sein kann,
$R^3$ für einen $C_{3-6}$-Alkyl-, einen $C_{3-7}$-Cycloalkyl-, einen gegebenenfalls wenigstens einfach substituierten Aryl- oder Heteroaryl-Rest oder für einen über eine $C_{1-3}$-Alkylen-Gruppe gebundenen, gegebenenfalls wenigstens einfach substituierten Aryl-oder Heteroaryl-Rest steht,
$R^4$ und $R^5$, gleich oder verschieden, für einen $C_{1-6}$-Alkyl-, einen $C_{3-7}$-Cycloalkyl-, einen Phenyl-, einen Benzyl- oder einen Phenethyl-Rest stehen oder $R^4$ und $R^5$ zusammen als $-(CH_2)_{3-6}-$ oder $-CH_2-CH_2-O-CH_2-CH_2-$ einen Ring bilden,
X und Y oder Y und Z zusammen für eine Bindung stehen,
A für einen gegebenenfalls wenigstens einfach substituierten Aryl- oder Heteroraryl-Rest steht,
in Form ihrer Racemate, Diasteromeren oder Enantiomeren als freie Base oder eines entsprechenden physiologisch verträglichen Salzes.

2. Substituierte 1-Aryl-but-3-enylamin- und 1-Aryl-but-2-enylaminverbindungen gemäß Anspruch 1, **dadurch gekennzeichnet, daß** $R^1$ und $R^2$ zusammen einen $(CH_2)_{2-6}$-Ring bilden, der auch mit wenigstens einem gegebenenfalls wenigstens einfach substituierten Aryl-Rest substituiert oder benzokondensiert sein kann.

3. Substituierte 1-Aryl-but-3-enytamin- und 1-Aryl-but-2-enylaminverbindungen gemäß Anspruch 1 oder 2, **dadurch gekennzeichnet, daß** $R^1$ und $R^2$ zusammen einen Cyclohexyl-Ring bilden, der auch mit einem gegebenenfalls wenigstens einfach substituierten Phenyl-Rest substituiert sein kann.

4. Substituierte 1-Aryl-but-3-enylamin- und 1-Aryl-but-2-enylaminverbindungen gemäß einem der Ansprüche 1 bis 3, **dadurch gekennzeichnet, daß** $R^3$ für einen gegebenenfalls wenigstens einfach substituierten Aryl-Rest oder für einen über eine $C_{1-3}$-Alkylen-Gruppe gebundenen, gegebenenfalls wenigstens einfach substituierten Aryl-Rest, vorzugweise für einen gegebenenfalls wenigstens einfach substituierten Phenyl-, Benzyl- oder Phenethyl-Rest steht.

5. Substituierte 1-Ary1-but-3-enylamin- und 1-Aryl-but-2-enylaminverbindungen gemäß einem der Ansprüche 1 bis 4, **dadurch gekennzeichnet, daß** die Reste $R^4$ und $R^5$, gleich oder verschieden, für einen $C_{1-6}$-Alkyl-Rest stehen oder zusammen einen $-(CH_2)_5-$ oder $-CH_2-CH_2-O-CH_2-CH_2-$Ring bilden, vorzugweise, gleich oder verschieden, für einen $C_{1-2}$-Alkyl-Rest stehen.

6. Substituierte 1-Aryl-but-3-enylamin- und 1-Aryl-but-2-enylaminverbindungen gemäß einem der Ansprüche 1 bis 5, **dadurch gekennzeichnet, daß** X und Y zusammen für eine Bindung stehen.

7. Substituierte 1-Aryl-but-3-enylamin- und 1-Aryl-but-2-enylaminverbindungen gemäß einem der Ansprüche 1 bis 6, **dadurch gekennzeichnet, daß** A für einen gegebenenfalls wenigstens einfach substituierten Aryl-Rest, vorzugsweise für einen gegebenenfalls wenigstens einfach substituierten Phenyl-Rest steht.

8. Substituierte 1-Aryl-but-3-enylamin- und 1-Aryl-but-2-enylaminverbindungen gemäß einem oder mehreren der Ansprüche 1 bis 7:

Dimethyl-[phenyl-(2-phenyl-cyclohex-1-enyl)-methyl]-amin,

{[2-(4-Chlor-phenyl)-cyclohex-1-enyl]-phenyl-methyl}-dimethyl-amin,
[(2-Benzyl-cyclohex-1-enyl)-phenyl-methyl]-dimethyl-amin,
{[2-(4-Fluor-3-methyl-phenyl)-cyclohex-1-enyl]-phenyl-methyl}-dimethyl-amin,
Dimethyl-[phenyl-(2-o-tolyl-cyclohex-1-enyl)-methyl]-amin,
[(2-Cyclopentyl-cyclohex-1-enyl)-phenyl-methyl]-dimethyl-amin,
Dimethyl-[phenyl-(2-m-tolyl-cyclohex-1-enyl)-methyl]-amin,
(Bicyclohexyl-1-en-2-yl-phenyl-methyl)-dimethyl-amin,
{[2-(4-Fluor-phenyl)-cyclohex-1-enyl]-phenyl-methyl)-dimethyl-amin,
Dimethyl-[(2-phenethyl-cyclohex-1-enyl)-phenyl-methyl]-amin,
{[2-(3-Methoxy-phenyl)-cyclohex-1-enyl]-phenyl-methyl}-dimethyl-amin,
Dimethyl-{phenyl-[2-(3-phenyl-propyl)-cyclohex-1-enyl]-methyl}-amin,
{[2-(2-Chlor-benzyl)-cyclohex-1-enyl]-phenyl-methyl}-dimethyl-amin,
{[2-(4-Fluor-benzyl)-cyclohex-1-enyl]-phenyl-methyl}-dimethyl-amin,
{[2-(3-Methoxy-benzyl)-cyclohex-1-enyl]-phenyl-methyl}-dimethyl-amin,
{[2-(3-Fluor-benzyl)-cyclohex-1-enyl]-phenyl-methyl}-dimethyl-amin,
{[2-(2-Methoxy-benzyl)-cyclohex-1-enyl]-phenyl-methyl}-dimethyl-amin,
{[2-(3,5-Difluor-benzyl)-cyclohex-1-enyl]-phenyl-methyl}-dimethyl-amin,
{[2-(2-Fluor-benzyl)-cyclohex-1-enyl]-phenyl-methyl}-dimethyl-amin,
{[2-(2-Chlor benzyl)-cyclohex-1-enyl]-phenyl-methyl}-dimethyl-amin,
{[2-(3-Fluor-benzyl)-cyclohex-1-enyl]-phenyl-methyl}-dimethyl-amin,
Dimethyl-{phenyl-[2-(3-trifluormethyl-benzyl)-cyclohex-1-enyl]-methyl}-amin,
Dimethyl-[(2-phenethyl-cyclohex-1-enyl)-phenyl-methyl]-amin,
3-[6-(Dimethylamino-phenyl-methyl)-cyclohex-1-enyl]-phenol,
Dimethyl-{phenyl-[2-(4-trifluormethylphenyl)-cyclohex-1-enyl]-methyl}-amin,
2-Chlor-5-[6-(dimethylamino-phenyl-methyl)-cyclohex-1-enyl]-phenol,
{[2-(4-Methoxy-phenylrcyclohex-2-enyl]-phenyl-methyl}-dimethyl-amin,
{[2-(4-Chlor-phenyl)-cyclohex-1-enyl]-phenyl-methyl}-dimethyl-amin,
Dimethyl-[(2-phenyl-cyclohex-1-enyl)-(4-trifluormethyl-phenyl)-methyl]-amin,

sowie die entsprechenden physiologisch verträglichen Salze, vorzugweise deren Hydrochloride.

**9.** Verfahren zur Herstellung von substituierten 1-Aryl-but-3-enylamin- und 1-Aryl-but-2-enylaminverbindungen der allgemeinen Formel I gemäß einem oder mehreren der Ansprüche 1 bis 8, **dadurch gekennzeichnet, daß** man wenigstens eine Mannich-Base der allgemeinen Formel II,

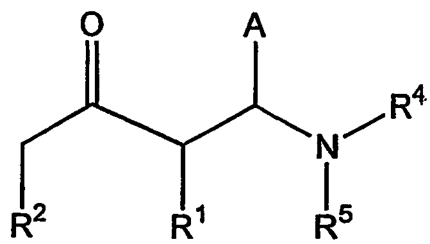

II

worin $R^1$, $R^2$, $R^4$, $R^5$ und A die Bedeutung gemäß der allgemeinen Formel I gemäß Anspruch 1 haben, mit wenigstens einer metallorganischen Verbindung der allgemeinen Formel $R^3$-B, worin B für MgCl, MgBr, Mgl oder Li steht und $R^3$ die Bedeutung gemäß der allgemeinen Formel I gemäß Anspruch 1 hat, zu wenigstens einem Alkohol der allgemeinem Formel III,

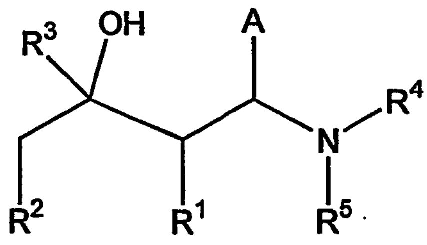

III

umsetzt, worin die Reste $R^1$ bis $R^5$ und A die Bedeutung gemäß der allgemeinen Formel I gemäß Anspruch 1 haben und diesen gegebenenfalls nach üblichen Methoden reinigt und/oder gegebenenfalls nach üblichen Methoden isoliert, und mit einer geeigneten Säure ggf. in Gegenwart eines geeigneten Lösungsmittels zu wenigstens einer Verbindung der allgemeinen Formel I gemäß Anspruch 1 umsetzt.

**10.** Verfahren gemäß Anspruch 9, **dadurch gekennzeichnet, daß** als geeignete Säure eine Protonensäure, eine Lewissäure oder eine deren Mischung eingesetzt wird.

**11.** Verfahren gemäß Anspruch 10, **dadurch gekennzeichnet, daß** als Protonensäure Bromwasserstoff, Chlorwasserstoff oder Ameisensäure eingesetzt wird.

**12.** Verfahren gemäß Anspruch 10 oder 11, **dadurch gekennzeichnet, daß** als Lewissäure Trimethylsilyliodid oder Chlortrimethylsilan eingesetzt wird.

**13.** Verfahren gemäß einem der Ansprüche 9 bis 12, **dadurch gekennzeichnet, daß** die Umsetzung des Alkohols mit der Säure bei einer Temperatur von 5 bis 150 °C, vorzugsweise bei einer Temperatur von 10 bis 130 °C, besonders bevorzugt bei einer Temperatur von 15 bis 120 °C durchgeführt wird.

**14.** Arzneimittel enthaltend wenigstens eine substituierte 1-Aryl-but-3-enylamin- oder 1-Aryl-but-2-enylaminverbindung gemäß einem der Ansprüche 1 bis 8 und gegebenenfalls physiologisch verträgliche Hilfsstoffe.

**15.** Arzneimittel gemäß Anspruch 14 zur Bekämpfung von Schmerzen.

**16.** Arzneimittel gemäß Anspruch 14 zur Behandlung von Depressionen.

**17.** Arzneimittel gemäß Anspruch 14 zur Behandlung von Hypotension.

**18.** Arzneimittel gemäß Anspruch 14 zur Behandlung von Hypertension.

**19.** Arzneimittel gemäß Anspruch 14 zur Behandlung von seniler Demenz.

**20.** Arzneimittel gemäß Anspruch 14 zur Behandlung von Morbus Alzheimer.

**21.** Arzneimittel gemäß Anspruch 14 zur Behandlung von allgemeinen kognitiven Dysfunktionen.

**22.** Arzneimittel gemäß Anspruch 14 zur Behandlung von Tinnitus.

**23.** Arzneimittel gemäß Anspruch 14 zur Behandlung von Schwerhörigkeit.

**24.** Arzneimittel gemäß Anspruch 14 zur Behandlung von Epilepsie.

**25.** Arzneimittel gemäß Anspruch 14 zur Behandlung von Fettsucht.

**26.** Arzneimittel gemäß Anspruch 14 zur Behandlung von Kachexie.

**27.** Arzneimittel gemäß Anspruch 14 zur Behandlung von Haminkontinenz.

28. Arzneimittel gemäß Anspruch 14 zur Anxiolyse.

29. Arzneimittel gemäß Anspruch 14 zur Diurese.

30. Verwendung wenigstens einer substituierten 1-Aryl-but-3-enylamin- oder 1-Aryl-but-2-enylaminverbindung gemäß einem der Ansprüche 1 bis 8 zur Herstellung eines Arzeimittels zur Bekämpfung von Schmerz, zur Behandlung von Depressionen, Hypotension, Hypertension, seniler Demenz, Morbus Alzheimer, allgemeinen kognitiven Dysfunktionen, Tinnitus, Schwerhörigkeit, Epilepsie, Fettsucht, Kachexie oder Haminkontinenz oder zur Anxiolyse oder Diurese.

## Claims

1. Substituted 1-aryl-but-3-enylamine and 1-aryl-but-2-enylamine compounds of the general formula I,

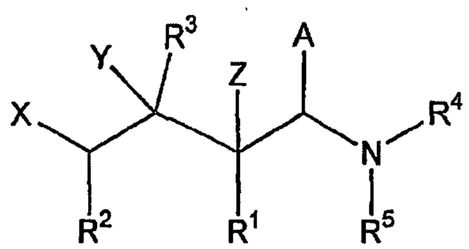

I

in which

$R^1$ and $R^2$, identical or different, denote a $C_{1-6}$ alkyl residue or together form a $(CH_2)_{2-6}$ ring, which may also be substituted or benzo-fused with at least one optionally at least mono-substituted aryl or heteroaryl residue,
$R^3$ denotes a $C_{3-6}$ alkyl, a $C_{3-7}$ cycloalkyl, an optionally at least mono-substituted aryl or heteroaryl residue or an optionally at least mono-substituted aryl or heteroaryl residue attached via a $C_{1-3}$ alkylene group,
$R^4$ and $R^5$, identical or different, denote a $C_{1-6}$ alkyl, a $C_{3-7}$ cycloalkyl, a phenyl, a benzyl or a phenethyl residue, or $R^4$ and $R^5$ together form a $(CH_2)_{3-6}$ or $-CH_2-CH_2-O-CH_2-CH_2$ ring,
X and Y or Y and Z together denote a bond,
A denotes an optionally at least mono-substituted aryl or heteroaryl residue,
in the form of the racemates, diastereomers or enantiomers thereof as a free base or a corresponding physiologically acceptable salt.

2. Substituted 1-aryl-but-3-enylamine and 1-aryl-but-2-enylamine compounds according to claim 1, **characterised in that** $R^1$ and $R^2$ together form a $(CH_2)_{2-6}$ ring, which may also be substituted or benzo-fused with at least one optionally at least mono-substituted aryl or heteroaryl residue.

3. Substituted 1-aryl-but-3-enylamine and 1-aryl-but-2-enylamine compounds according to claim 1 or 2, **characterised in that** $R^1$ and $R^2$ together form a cyclohexyl ring, which may also be substituted with an optionally at least mono-substituted phenyl residue.

4. Substituted 1-aryl-but-3-enylamine and 1-aryl-but-2-enylamine compounds according to one of claims 1 to 3, **characterised in that** $R^3$denotes an optionally at least mono-substituted aryl residue or an optionally at least mono-substituted aryl residue attached via a $C_{1-3}$ alkylene group, preferably an optionally at least mono-substituted phenyl, benzyl or phenethyl residue.

5. Substituted 1-aryl-but-3-enylamine and 1-aryl-but-2-enylamine compounds according to one of claims 1 to 4, **characterised in that** the residues $R^4$ and $R^5$, identical or different, denote a $C_{1-6}$ alkyl residue or together form a $-(CH_2)_5-$ or $-CH_2-CH_2-O-CH_2-CH_2$ ring, preferably, identical or different, denote a $C_{1-2}$ alkyl residue.

6. Substituted 1-aryl-but-3-enylamine and 1-aryl-but-2-enylamine compounds according to one of claims 1 to 5, **characterised in that** X and Y together denote a bond.

7. Substituted 1-aryl-but-3-enylamine and 1-aryl-but-2-enylamine compounds according to one of claims 1 to 6, **characterised in that** A denotes an optionally at least mono-substituted aryl residue, preferably an optionally at least mono-substituted phenyl residue.

8. Substituted 1-aryl-but-3-enylamine and 1-aryl-but-2-enylamine compounds according to one or more of claims 1 to 7:

Dimethyl-[phenyl-(2-phenyl-cyclohex-1-enyl)-methyl]-amine,
{[2-(4-Chloro-phenyl)-cyclohex-1-enyl]-phenyl-methyl}-dimethyl-amine,
[(2-Benzyl-cyclohex-1-enyl)-phenyl-methyl]-dimethylamine,
{[2-(4-Fluoro-3-methyl-phenyl)-cyclohex-1-enyl]-phenyl-methyl}-dimethyl-amine,
Dimethyl-[phenyl-(2-o-tolyl-cyclohex-1-enyl)-methyl]-amine,
[(2-Cyclopentyl-cyclohex-1-enyl)-phenyl-methyl]-dimethyl-amine,
Dimethyl-[phenyl-(2-m-tolyl-cyclohex-1-enyl)-methyl]-amine,
(Bicyclohexyl-1-en-2-yl-phenyl-methyl)-dimethyl-amine,
{[2-(4-Fluoro-phenyl)-cyclohex-1-enyl]-phenyl-methyl}-dimethyl-amine,
Dimethyl-[(2-phenethyl-cyclohex-1-enyl)-phenylmethyl]-amine,
{[2-(3-Methoxy-phenyl)-cyclohex-1-enyl]-phenylmethyl}-dimethyl-amine,
Dimethyl-{phenyl-[2-(3-phenyl-propyl)-cyclohex-1-enyl]-methyl}-amine,
{[2-(2-Chloro-benzyl)-cyclohex-1-enyl]-phenyl-methyl}-dimethyl-amine,
{[2-(4-Fluoro-benzyl)-cyclohex-1-enyl]-phenyl-methyl}-dimethyl-amine,
{[2-(3-Methoxy-benzyl)-cyclohex-1-enyl]-phenylmethyl}-dimethyl-amine,
{[2-(3-Fluoro-benzyl)-cyclohex-1-enyl]-phenyl-methyl}-dimethyl-amine,
{[2-(2-Methoxy-benzyl)-cyclohex-1-enyl]-phenylmethyl}-dimethyl-amine,
([2-(3,5-Difluoro-benzyl)-cyclohex-1-enyl]-phenylmethyl}-dimethyl-amine,
{[2-(2-Fluoro-benzyl)-cyclohex-1-enyl]-phenyl-methyl}-dimethyl-amine,
{[2-(2-Chloro-benzyl)-cyclohex-1-enyl]-phenyl-methyl}-dimethyl-amine,
{[2-(3-Fluoro-benzyl)-cyclohex-1-enyl]-phenyl-methyl}-dimethyl-amine,
Dimethyl-{phenyl-[2-(3-trifluoromethyl-benzyl)-cyclohex-1-enyl]-methyl}-amine,
Dimethyl-[(2-phenethyl-cyclohex-1-enyl)-phenylmethyl}-amine,
3-[6-(Dimethylamino-phenyl-methyl)-cyclohex-1-enyl]-phenol,
Dimethyl-{phenyl-(2-(4-trifluoromethylphenyl)-cyclohex-1-enyl]-methyl}-amine,
2-Chloro-5-[6-(dimethylamino-phenyl-methyl)-cyclohex-1-enyl]-phenol,
{[2-(4-Methoxy-phenyl)-cyclohex-2-enyl]-phenylmethyl}-dimethyl-amine,
{[2-(4-Chloro-phenyl)-cyclohex-1-enyl]-phenyl-methyl}-dimethyl-amine,
Dimethyl-[(2-phenyl-cyclohex-1-enyl)-(4-trifluoromethyl-phenyl)-methyl]-amine,

and the corresponding physiologically acceptable salts, preferably the hydrochlorides thereof.

9. A process for the production of substituted 1-aryl-but-3-enylamine and 1-aryl-but-2-enylamine compounds of the general formula I according to one or more of claims 1 to 8, **characterised in that** at least one Mannich base of the general formula II,

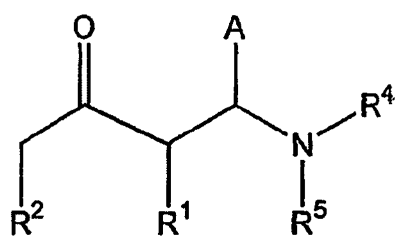

II

in which $R^1$, $R^2$, $R^4$, $R^5$ and A have the meaning according to the general formula I according to claim 1, is reacted

with at least one organometallic compound of the general formula $R^3$-B, in which B denotes MgCl, MgBr, MgI or Li and $R^3$ has the meaning according to the general formula I according to claim 1, to yield at least one alcohol of the general formula III,

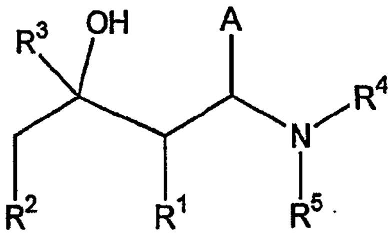

III

in which the residues $R^1$ to $R^5$ and A have the meaning according to the general formula I according to claim 1, and this is optionally purified by conventional methods and/or optionally isolated by conventional methods, and reacted with a suitable acid optionally in the presence of a suitable solvent to yield at least one compound of the general formula I according to claim 1.

**10.** A process according to claim 9, **characterised in that** a protonic acid, a Lewis acid or a mixture thereof is used as suitable acid.

**11.** A process according to claim 10, **characterised in that** hydrogen bromide, hydrogen chloride or formic acid is used as protonic acid.

**12.** A process according to claim 10 or 11, **characterised in that** trimethylsilyl iodide or chlorotrimethylsilane is used as the Lewis acid.

**13.** A process according to one of claims 9 to 12, **characterised in that** reaction of the alcohol with the acid is performed at a temperature of 5 to 150°C, preferably at a temperature of 10 to 130°C, particularly preferably at a temperature of 15 bis 120°C.

**14.** A pharmaceutical preparation containing at least one substituted 1-aryl-but-3-enylamine or 1-aryl-but-2-enylamine compound according to one of claims 1 to 8 and optionally physiologically acceptable auxiliary substances.

**15.** A pharmaceutical preparation according to claim 14 for combatting pain.

**16.** A pharmaceutical preparation according to claim 14 for the treatment of depression.

**17.** A pharmaceutical preparation according to claim 14 for the treatment of hypotension.

**18.** A pharmaceutical preparation according to claim 14 for the treatment of hypertension.

**19.** A pharmaceutical preparation according to claim 14 for the treatment of senile dementia.

**20.** A pharmaceutical preparation according to claim 14 for the treatment of Alzheimer's disease.

**21.** A pharmaceutical preparation according to claim 14 for the treatment of general cognitive dysfunction.

**22.** A pharmaceutical preparation according to claim 14 for the treatment of tinnitus.

**23.** A pharmaceutical preparation according to claim 14 for the treatment of hardness of hearing.

**24.** A pharmaceutical preparation according to claim 14 for the treatment of epilepsy.

**25.** A pharmaceutical preparation according to claim 14 for the treatment of obesity.

**26.** A pharmaceutical preparation according to claim 14 for the treatment of cachexia.

**27.** A pharmaceutical preparation according to claim 14 for the treatment of urinary incontinence.

**28.** A pharmaceutical preparation according to claim 14 for anxiolysis.

**29.** A pharmaceutical preparation according to claim 14 for diuresis.

**30.** Use of at least one substituted 1-aryl-but-3-enylamine or 1-aryl-but-2-enylamine compound according to one of claims 1 to 8 for the production of a pharmaceutical preparation for combatting pain, for the treatment of depression, hypotension, hypertension, senile dementia, Alzheimer's disease, general cognitive dysfunction, tinnitus, hardness of hearing, epilepsy, obesity, cachexia or urinary incontinence or for anxiolysis or diuresis.

## Revendications

**1.** Composés substitués de 1-aryl-but-3-énylamine et de 1-aryl-but-2-énylamine de formule générale I,

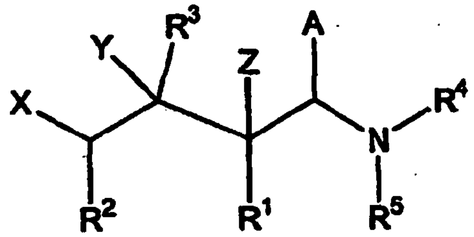

I

dans laquelle

$R^1$ et $R^2$ sont identiques ou différents et représentent un radical $C_{1-6}$-alkyle ou forment ensemble, sous forme de $(CH_2)_{2-6}$ un cycle qui peut également être substitué par au moins un radical aryle ou hétéroaryle le cas échéant au moins monosubstitué ou être benzocondensé,
$R^3$ représente un radical $C_{3-6}$-alkyle, $C_{3-7}$-cycloalkyle, un radical aryle ou hétéroaryle le cas échéant au moins monosubstitué ou un radical aryle ou hétéroaryle lié via un groupe $C_{1-3}$-alkylène, le cas échéant au moins monosubstitué,
$R^4$ et $R^5$ sont identiques ou différents et représentent un radical $C_{1-6}$-alkyle, $C_{3-7}$-cycloalkyle, phényle, benzyle ou phénéthyle ou $R^4$ et $R^5$ forment ensemble, sous forme de - $(CH_2)_{3-6}$- ou $-CH_2-CH_2-O-CH_2-CH_2-$, un cycle,
X et Y ou Y et Z représentent ensemble une liaison,
A représente un radical aryle ou hétéroaryle le cas échéant au moins monosubstitué,
sous forme de leurs racémates, diastéréo-isomères ou énantiomères, sous forme de base libre ou d'un sel physiologiquement acceptable correspondant.

**2.** Composés substitués de 1-aryl-but-3-énylamine et de 1-aryl-but-2-énylamine selon la revendication 1, **caractérisés en ce que** $R^1$ et $R^2$ forment ensemble un cycle $(CH_2)_{2-6}$, qui peut également être substitué par au moins un radical aryle le cas échéant au moins monosubstitué ou être benzocondensé.

**3.** Composés substitués de 1-aryl-but-3-énylamine et de 1-aryl-but-2-énylamine selon la revendication 1 ou 2, **caractérisés en ce que** $R^1$ et $R^2$ forment ensemble un cycle cyclohexyle, qui peut également être substitué par un radical phényle le cas échéant au moins monosubstitué.

**4.** Composés substitués de 1-aryl-but-3-énylamine et de 1-aryl-but-2-énylamine selon l'une quelconque des revendications 1 à 3, **caractérisés en ce que** $R^3$ représente un radical aryle le cas échéant au moins monosubstitué ou un radical aryle lié via un groupe $C_{1-3}$-alkylène, le cas échéant au moins monosubstitué, de préférence un radical phényle, benzyle ou phénéthyle, le cas échéant au moins monosubstitué.

**5.** Composés substitués de 1-aryl-but-3-énylamine et de 1-aryl-but-2-énylamine selon l'une quelconque des revendications 1 à 4, **caractérisés en ce que** les radicaux $R^4$ et $R^5$ sont identiques ou différents et représentent un radical $C_{1-6}$-alkyle ou forment ensemble un cycle $-(CH_2)_5-$ ou $-CH_2-CH_2-O-CH_2-CH_2-$, et sont de préférence identiques ou différents et représentent un radical $C_{1-2}$-alkyle.

**6.** Composés substitués de 1-aryl-but-3-énylamine et de 1-aryl-but-2-énylamine selon l'une quelconque des revendications 1 à 5, **caractérisés en ce que** X et Y représentent ensemble une liaison.

**7.** Composés substitués de 1-aryl-but-3-énylamine et de 1-aryl-but-2-énylamine selon l'une quelconque des revendications 1 à 6, **caractérisés en ce que** A représente un radical aryle le cas échéant au moins monosubstitué, de préférence un radical phényle le cas échéant au moins monosubstitué.

**8.** Composés substitués de 1-aryl-but-3-énylamine et de 1-aryl-but-2-énylamine selon l'une ou plusieurs des revendications 1 à 7 :

diméthyl-[phényl-(2-phénylcyclohex-l-ényl)-méthyl]-amine,
{[2-(4-chlorophényl)-cyclohex-1-ényl]-phénylméthyl}-diméthylamine,
[(2-benzylcyclohex-1-ényl)-phénylméthyl]-diméthylamine,
{[2-(4-fluoro-3-méthylphényl)-cyclohex-1-ényl]-phénylméthyl}-diméthylamine,
diméthyl-[phényl-(2-o-tolylcyclohex-1-ényl)-méthyl]-amine,
[(2-cyclopentyl-cyclohex-1-ényl)-phénylméthyl]-diméthylamine,
diméthyl-[phényl-(2-m-tolyl-cyclohex-1-ényl)-méthyl]-amine,
(bicyclohexyl-1-en-2-yl-phénylméthyl)-diméthylamine, {[2-(4-fluorophényl)-cyclohex-1-ényl]-phénylméthyl}-diméthylamine,
diméthyl-[(2-phénéthyl-cyclohex-1-ényl)-phénylméthyl]-amine,
{[2-(3-méthoxyphényl)-cyclohex-1-ényl]-phénylméthyl}-diméthylamine,
diméthyl-{phényl-[2-(3-phénylpropyl)-cyclohex-1-ényl]-méthyl}-amine,
{[2-(2-chlorobenzyl)-cyclohex-1-ényl]-phénylméthyl}-diméthylamine,
{[2-(4-fluorobenzyl)-cyclohex-1-ényl]-phénylméthyl}-diméthylamine,
{[2-(3-méthoxybenzyl)-cyclohex-1-ényl]-phénylméthyl}-diméthylamine,
{[2-(3-fluorobenzyl)-cyclohex-1-ényl]-phénylméthyl}-diméthylamine,
{[2-(2-méthoxybenzyl)-cyclohex-1-ényl]-phénylméthyl}-diméthylamine,
{[2-(3,5-difluorobenzyl)-cyclohex-1-ényl]-phénylméthyl)-diméthylamine,
{[2-(2-fluorobenzyl)-cyclohex-1-ényl]-phénylméthyl}-diméthylamine,
{[2-(2-chlorobenzyl)-cyclohex-1-ényl]-phénylméthyl}-diméthylamine,
{[2-(3-fluorobenzyl)-cyclohex-1-ényl]-phénylméthyl}-diméthylamine,
diméthyl-{phényl-[2-(3-trifluorométhyl-benzyl)-cyclohex-1-ényl]-méthyl}-amine,
diméthyl-[(2-phenéthyl-cyclohex-1-ényl)-phénylméthyl]-amine,
3[6-(diméthylaminophénylméthyl)-cyclohex-1-ényl]-phénol,
diméthyl-{phényl-[2-(4-trifluorométhylphényl)-cyclohex-1-ényl]-méthyl}-amine,
2-chloro-5-[6-(diméthylaminophénylméthyl)-cyclohex-1-ényl]-phénol,
{[2-(4-méthoxyphényl)-cyclohex-2-ényl]-phénylméthyl}-diméthylamine,
{[2-(4-chlorophényl)-cyclohex-1-ényl]-phénylméthyl}-diméthylamine,
diméthyl-[(2-phényl-cyclohex-1-ényl)-(4-trifluorométhylphényl)-méthyl]amine,

ainsi que les sels physiologiquement acceptables correspondants, de préférence leurs chlorhydrates.

**9.** Procédé pour la préparation de composés substitués de 1-aryl-but-3-énylamine et de 1-aryl-but-2-énylamine de formule générale I selon l'une ou plusieurs des revendications 1 à 8, **caractérisé en ce qu'**on transforme au moins une base de Mannich de formule générale II,

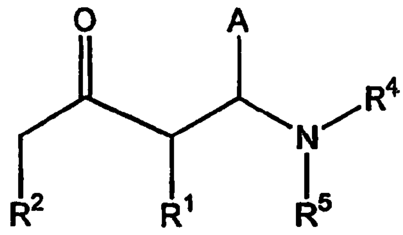

II

dans laquelle $R^1$, $R^2$, $R^4$, $R^5$ et A ont la signification selon la formule générale I selon la revendication 1, avec au moins un composé métallo-organique de formule générale $R^3$-B, dans laquelle B représente MgCl, MgBr, MgI ou Li et $R^3$ a la signification selon la formule générale I selon la revendication 1, en au moins un alcool de formule générale III,

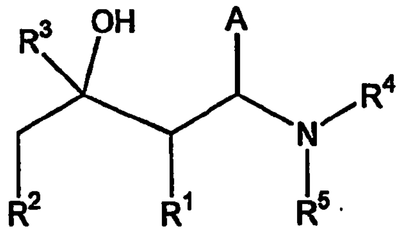

III

dans laquelle les radicaux $R^1$ à $R^5$ et A ont la signification selon la formule générale I selon la revendication 1 et on le purifie le cas échéant selon des procédés usuels et/ou on l'isole le cas échéant selon des procédés usuels et on le transforme avec un acide approprié, le cas échéant en présence d'un solvant approprié, en au moins un composé de formule générale I selon la revendication 1.

**10.** Procédé selon la revendication 9, **caractérisé en ce qu'**on utilise comme acide approprié un acide protonique, un acide de Lewis ou un mélange de ceux-ci.

**11.** Procédé selon la revendication 10, **caractérisé en ce qu'**on utilise comme acide protonique de l'acide bromhydrique, de l'acide chlorhydrique ou de l'acide formique.

**12.** Procédé selon la revendication 10 ou 11,
**caractérisé en ce qu'**on utilise comme acide de Lewis de l'iodure de triméthylsilyle ou du chlorotriméthylsilane.

**13.** Procédé selon l'une quelconque des revendications 9 à 12, **caractérisé en ce que** la transformation de l'alcool avec l'acide est réalisée à une température de 5 à 150°C, de préférence à une température de 10 à 130°C, de manière particulièrement préférée à une température de 15 à 120°C.

**14.** Médicament contenant au moins un composé substitué de 1-aryl-but-3-énylamine ou de 1-aryl-but-2-énylamine selon l'une quelconque des revendications 1 à 8 et le cas échéant des adjuvants physiologiquement acceptables.

**15.** Médicament selon la revendication 14 destiné à lutter contre des douleurs.

**16.** Médicament selon la revendication 14 destiné au traitement de dépressions.

**17.** Médicament selon la revendication 14 destiné au traitement de l'hypotension.

**18.** Médicament selon la revendication 14 destiné au traitement de l'hypertension.

**19.** Médicament selon la revendication 14 destiné au traitement de la démence sénile.

**20.** Médicament selon la revendication 14 destiné au traitement de la maladie d'Alzheimer.

**21.** Médicament selon la revendication 14 destiné au traitement des dysfonctionnements cognitifs généraux.

**22.** Médicament selon la revendication 14 destiné au traitement des acouphènes.

**23.** Médicament selon la revendication 14 destiné au traitement de la surdité.

**24.** Médicament selon la revendication 14 destiné au traitement de l'épilepsie.

**25.** Médicament selon la revendication 14 destiné au traitement de l'obésité.

**26.** Médicament selon la revendication 14 destiné au traitement de la cachexie.

**27.** Médicament selon la revendication 14 destiné au traitement de l'incontinence urinaire.

**28.** Médicament selon la revendication 14 destiné à l'anxiolyse.

**29.** Médicament selon la revendication 14 destiné à la diurèse.

**30.** Utilisation d'au moins un composé substitué de la 1-aryl-but-3-énylamine ou de la 1-aryl-but-2-énylamine selon l'une quelconque des revendications 1 à 8 pour la préparation d'un médicament destiné à lutter contre la douleur, au traitement de dépressions, de l'hypotension, de l'hypertension, de la démence sénile, de la maladie d'Alzheimer, des dysfonctionnements cognitifs généraux, des acouphènes, de la surdité, de l'épilepsie, de l'obésité, de la cachexie ou de l'incontinence urinaire ou à l'anxiolyse ou à la diurèse.